# EUROPÄISCHE PATENTSCHRIFT

(11) **EP 2 263 904 B1**
(45) Veröffentlichungstag und Bekanntmachung des Hinweises auf die Patenterteilung: **03.08.2011**
(21) Anmeldenummer: 09162775.2
(22) Anmeldetag: 16.06.2009
(51) Int. Cl.: B60K 28/06, G01N 33/497

(54) **Alkohol-Interlock-System mit drahtloser Datenübertragungs- und Sicherheitsfunktion**
Alcohol interlock system with wireless data transfer and security function
Système de verrouillage d'alcool doté d'une fonction de transmission de données sans fil et de sécurité

(43) Veröffentlichungstag der Anmeldung: 22.12.2010
(73) Patentinhaber: Dräger Safety AG & Co. KGaA, 23560 Lübeck (DE)
(72) Erfinder: Dr,Morley, Stefan, 23556, Lübeck (DE); Zimmermann, Martin, 22083, Hamburg (DE); Reinhart, Michael, 23617, Stockelsdorf (DE)
(74) Vertreter: UEXKÜLL & STOLBERG

(56) Entgegenhaltungen:
- WO-A-2009/048809
- DE-A1- 19 742 261
- US-A1- 2006 202 842
- US-A1- 2007 273 537
- US-B1- 6 748 792
- US-B1- 7 256 700

## Beschreibung

Die vorliegende Erfindung betrifft eine Vorrichtung, die ausgestaltet ist, um das Starten eines Fahrzeugs oder die Bedienung einer Maschine durch einen alkoholisierten Benutzer zu verhindern. Eine solche Vorrichtung wird üblicherweise als Alkohol-Interlock-System, kurz "Interlock", bezeichnet und verkörpert allgemein die Kombination aus einem Alkoholmessgerät sowie einer Wegfahrsperre für ein Kraftfahrzeug bzw. einer Blockiereinrichtung für eine Maschine.

Üblicherweise wird ein Interlock-System verwendet, um einen alkoholisierten Fahrer nach einer positiven Alkoholmessung daran zu hindern, den Motor eines Fahrzeugs (PKW, LKW, Bus, etc.) zu starten. Dazu beinhaltet ein Interlock-System im Wesentlichen zwei Komponenten: ein Alkoholmessgerät, das sich in der Regel im Inneren des Fahrzeugs befindet, sowie ein mit dem Alkoholmessgerät gekoppeltes Steuergerät, das beispielsweise unter dem Armaturenbrett des Fahrzeugs fest installiert und ausgestaltet ist, um die Stromzufuhr zum Anlasser des Fahrzeugs freizuschalten oder zu blockieren. Das Alkoholmessgerät ist vorzugsweise ein Atemalkoholmessgerät, das als ein Handgerät ausgestaltet und über ein elektrisches Verbindungskabel mit dem Steuergerät verbunden ist. Alternativ kann das Handgerät auch drahtlos mit dem Steuergerät gekoppelt sein (beispielsweise über Infrarot, Bluetooth oder ähnliche Protokolle).

Ein solches Interlock-System funktioniert üblicherweise wie folgt: Nach dem Einschalten der Zündung des Fahrzeugs fordert das Interlock-System den Fahrer zur Abgabe einer Atemprobe auf. Das Messergebnis der gemessenen Atemalkoholkonzentration, aus der sich mittels bekannter Algorithmen der Alkoholpegel bestimmen lässt, entscheidet darüber, ob der Anlasser des Fahrzeugs freigeschaltet wird und der Motor gestartet werden kann.

Grundsätzlich besteht bei Anwendung des Interlock-Systems die Möglichkeit der Manipulation, indem beispielsweise eine angeforderte Alkoholmessung durch eine zweite Person durchgeführt wird, die nicht der zu überprüfende Fahrer ist. Um eine solche Umgehungsmöglichkeit auszuschließen, weisen bekannte Interlock-Systeme üblicherweise eine Wiederholungsfunktion der Alkoholmessung auf. Das heißt, das Interlock-System fordert den Fahrer nach einem zufällig generierten Zeitintervall erneut auf, eine Alkoholmessung durchzuführen.

Zur Bestimmung der Atemalkoholkonzentration bzw. des Alkoholpegels beinhaltet das Messsystem eines Interlock-Systems normalerweise ein elektrochemisches Sensorsystem, wie es auch in Atemalkoholmessgeräten der Polizei verwendet wird. Dieses Sensorsystem spricht sehr spezifisch auf Alkohol an, so dass andere ausgeatmete Substanzen oder Zigarettenrauch das Messergebnis nicht verfälschen.

Während der Benutzung des Fahrzeugs werden in einem Datenspeicher des Interlock-System alle für den Einsatz relevanten Ereignisse aufgezeichnet, wie zum Beispiel Datum, Uhrzeit, Abgabe oder Verweigerung einer Atemprobe, gemessene Alkoholkonzentration, Motorstarts- und Motorstops, elektrisches Überbrücken des Interlock-Systems sowie andere Manipulationsversuche. Diese Daten können zu einem Protokoll zusammengestellt und zum Beispiel mit Hilfe eines Datenkabels, durch das das Handgerät des Interlock-Systems mit einem externen Computer verbunden wird, ausgelesen werden. Das Auslesen kann beispielsweise in einer autorisierten Werkstatt oder vor Ort durch einen autorisierten Monteur erfolgen.

Die DE 197 42 261 A1 beschreibt eine Vorrichtung zum Blockieren der Bedienung eines Fahrzeugs durch einen alkoholisierten Fahrer. Dabei ist das Alkoholmessgerät ausgestaltet, um an einem Körperteil (Arm oder Bein) des Fahrers befestigt zu werden und um den Alkoholpegel des Fahrers mit Hilfe eines elektrochemischen Gassensors über die Hautpermeation messen zu können. Die eigentliche Auswerteeinheit der Vorrichtung, über die eine Freigabe bzw. ein Blockieren des Fahrzeugs erfolgt, ist fest in dem Fahrzeug montiert und kommuniziert drahtlos mit dem Messgerät.

Die WO 2009/048809 A1, die den Oberbegriff des Anspruchs 1 bildet, beschreibt ein Interlock-System mit einem Mittel für eine biometrische Prüfung zur Identifikation einer Person, wobei die biometrischen Daten mit von der Person hinterlegen Daten in einer Datenbank verglichen werden. Ferner weist das Interlock-System ein Modul für eine wechselseitige Datenübertragung auf, welches für eine Fernabfrage des Zustandes des Interlock-Systems dient.

Die US 6,748,792 B1 offenbart eine Vorrichtung zur Erkennung einer korrekten Anwendung eines Alkoholmessgerätes umfassend ein Atemalkoholmessgerät und eine Kamera, wobei die Kamera in dem Alkoholmessgerät zur Aufnahme der Testperson angeordnet ist. Ferner ist die Kamera mit einem Lagesensor ausgeführt, um eine verwertbare Aufnahme zur Identifikation der Testperson zu erzielen. Ferner können die Aufnahmen kabellos für eine Auswertung versendet werden.

Die US 7,256,700 B1 betrifft ein Interlock-System, mit Hilfe dessen das Starten eines Fahrzeugs durch einen alkoholisierten Fahrer verhindert wird. Das Interlock-System ist mit einem Mobiltelefon oder einer ähnlichen Kommunikationseinrichtung gekoppelt, mit der eine fehlgeschlagene Alkoholmessung kommuniziert wird, indem über das Mobiltelefon eine Sprachmitteilung versendet wird. Ferner können über das Mobiltelefon auch Daten versendet werden, die in dem Interlock-System gespeichert sind.

Die US 2007/0273537 A1 offenbart ein kombiniertes Test- und Lokalisierungssystem, das unter anderem ein Interlock-System beinhaltet. Das Interlock-System dient dabei auf bekannte Weise dazu, das Starten eines Fahrzeugs durch einen alkoholisierten Fahrer zu verhindern. Das System ist ferner mit einem EMHA-System (Electronic Monitoring Home Arrest) ausgestattet, das beispielsweise über ein Mobiltelefon mit einem entfernten Server kommunizieren kann. Über dieses Mobiltelefon können aber auch in dem Interlock-System gespeicherte Daten an den Server übertragen werden.

Ein Nachteil der bekannten Interlock-Systeme besteht darin, dass sie nicht von einer entfernt gelegenen Zentrale deaktiviert bzw. reaktiviert werden können. Viele Interlock-Systeme werden nicht dazu verwendet, um einen zum Beispiel aufgrund eines Alkoholdelikts verurteilten Straftäter daran zu hindern, unter Alkoholeinfluss sein Auto zu starten. Vielmehr sind viele Interlock-Systeme auf freiwilliger Basis im Einsatz oder dienen zur Erhöhung der Sicherheit zum Beispiel in Bereichen des Schwerguttransports, des Gefahrenguttransports, in Schulbussen, in Reisebussen oder auch im öffentlichen oder privaten Personennahverkehr. Sollte zum Beispiel aufgrund eines technischen Defekts des Interlock-Systems oder aufgrund einer Fehlfunktion bzw. einer Fehlerfassung des Alkoholsensors über verbotene Parameter von weiteren sensors das Starten des Fahrzeugs verhindert werden, so ist der Fahrer, auch wenn er nicht unter Alkoholeinfluss steht, nicht in der Lage, sein Fahrzeug zu starten.

Wie vorstehend erläutert, wird der Fahrer eines Fahrzeugs üblicherweise nicht nur zu Beginn einer Fahrt, sondern auch während der Fahrt, vom Interlock-System in unregelmäßigen und zufällig bestimmten Zeitabständen aufgefordert, einen Alkoholtest durchzuführen. Bei einer plötzlichen Fehlfunktion des Interlock-Systems kann es daher passieren, dass sich beispielsweise ein Gefahrengut-Transporter mitten auf der Autobahn nicht mehr starten lässt. Eine Freischaltung bzw. Deaktivierung des defekten Interlock-Systems kann in einem solchen Fall nur vor Ort am Interlock-System des Fahrzeugs selbst durchgeführt werden, indem ein speziell geschulter und autorisierter Monteur zu dem "stehen gebliebenen" Fahrzeug fährt und zum Beispiel mit Hilfe eines Computers, der über ein Datenkabel mit einer entsprechenden Schnittstelle des Interlock-Systems verbunden wird, einen geheimen Freischalt-Code in das Interlock-System eingibt, wodurch das Interlock-System freigeschaltet bzw. deaktiviert wird und sich der Motor des Fahrzeugs wieder starten lässt, ohne dass eine erneute Alkoholkontrolle durchgeführt wird. (Dies ist jedoch nur möglich, wenn kein Hardware-Defekt vorliegt, der eine Kommunikation mit dem Interlock gänzlich unmöglich macht.) Anschließend muss das Interlock-System in einer Werkstatt repariert und reaktiviert werden. Sollte kein Monteur verfügbar sein, so muss das Fahrzeug zu einer autorisierten Reparaturwerkstatt abgeschleppt werden, um dort das Interlock-System freischalten bzw. deaktivieren zu lassen. Beide Lösungen sind sehr zeitaufwendig und führen zu hohen Folgekosten.

Der vorliegenden Erfindung liegt daher die Aufgabe zugrunde, ein Interlock-System zur Verfügung zu stellen, mit Hilfe dessen die vorstehend genannten Nachteile überwunden werden. Es ist insbesondere Aufgabe der Erfindung, ein Interlock-System zur Verfügung zu stellen, das ausgestaltet ist, um das Starten eines Fahrzeugs oder die Bedienung einer Maschine durch einen alkoholisierten Fahrer/Benutzer zu verhindern, und das ausgestaltet ist, um im Interlock-System gespeicherte Daten drahtlos zu einem entfernt gelegenen Kontroll-System zu übertragen und/oder um Daten von einem entfernt gelegenen Kontroll-System drahtlos zum Interlock-System zu übertragen.

Eine weitere Aufgabe der vorliegenden Erfindung besteht darin, das Interlock-System von einem entfernt gelegenen Kontroll-System aus drahtlos zu deaktivieren (freizuschalten bzw. zu überbrücken) und/oder zu reaktivieren.

Diese und weitere Aufgaben werden durch ein Interlock-System mit den Merkmalen des Patentanspruchs 1 gelöst. In den abhängigen Ansprüchen sind vorteilhafte und bevorzugte Weiterbildungen des erfindungsgemäßen Interlock-Systems angegeben.

Wie vorstehend erläutert, ist ein Interlock-System ein Alkoholmessgerät mit Wegfahrsperre. Die Alkoholmessung wird üblicherweise als Atemalkoholmessung durchgeführt, wobei dies aber keine notwendige Bedingung für das erfindungsgemäße Interlock-System ist. Prinzipiell sind für das erfindungsgemäße Interlock-System auch andere Verfahren zur Alkoholmessung einsetzbar (so zum Beispiel eine Messung der Alkoholkonzentration über die Hautpermeation). Das Ziel des erfindungsgemäßen Interlock-Systems besteht darin, einen alkoholisierten Fahrer daran zu hindern, den Motor des Fahrzeugs zu starten, in dem das Interlock-System eingebaut ist. Durch den Einbau des Interlock-Systems können alkoholbedingte Unfälle vermieden werden. Ferner ist das Interlock-System geeignet, langfristige Verhaltensänderungen des Fahrers im Umgang mit Alkohol zu unterstützen.

Die praktische Verfahrensweise ist dabei im Allgemeinen so, dass der Fahrer des Fahrzeugs beim Versuch das Fahrzeug zu starten vom Interlock-System aufgefordert wird, eine Atemalkoholmessung durchzuführen. Abhängig vom Ergebnis dieser Messung (d.h. Alkoholpegel) wird dann die Signalkette zum Anlasser des Motors freigegeben und dem Fahrer das Starten des Motors ermöglicht.

Alle relevanten Vorgänge im Zusammenhang mit der Benutzung des Interlock-Systems in einem Fahrzeug werden in einem Datenspeicher des Interlock-Systems abgelegt. Dieser Speicher kann zu einem beliebigen Zeitpunkt von dazu berechtigten Personen bzw. Institutionen ausgelesen werden. Das Auslesen des Datenspeichers erfolgt zum Beispiel im Rahmen von Programmen für Trunkenheitsfahrer in regelmäßigen Abständen (z.B. 1x/Monat) in autorisierten Servicewerkstätten. Die Daten werden dann von einer Datenmanagementsoftware verarbeitet, und anschließend werden die relevanten Informationen an eine überwachende Stelle (z.B. Bewährungshelfer) übermittelt (z.B. per Email, SMS, Mobiltelefon oder Fax).

Es besteht weiterhin die Möglichkeit, bei Auftreten bestimmter Ereignisse (z.B. nicht bestandener Atemtest) die "Service Periode" zu verkürzen, um somit einen vorzeitigen Besuch einer Servicewerkstatt zu erzwingen.

Grundsätzlich kann ein technischer Defekt des Interlock-Systems dazu führen, dass das Fahrzeug nicht mehr gestartet werden kann. In diesem Fall muss das Interlock-System in der Regel überbrückt werden. Dies kann bedeuten, dass dazu das Fahrzeug in eine Servicewerkstatt abgeschleppt werden muss oder dass ein Techniker zu dem stehen gebliebenen Fahrzeug fahren muss, um den Defekt zu beheben oder die Interlock-Funktionalität zu überbrücken.

Wie vorstehend erläutert, kann der Datenspeicher von bekannten Interlock-Systemen nur in regelmäßigen Abständen, zum Beispiel in einer Servicewerkstatt, ausgelesen werden. Daher besteht ein zeitlicher Verzug (zum Beispiel 4 Wochen) zwischen der Registrierung von Fehlverhalten im Datenspeicher und der Information von überwachenden Stellen oder Aufsichtsbehörden. Der Vorteil des Interlock-Systems der vorliegenden Erfindung besteht darin, dass dieser zeitliche Verzug verkürzt werden kann, um so eine schnelle Reaktion auf ein Fehlverhalten des Fahrers zu ermöglichen und/oder um einer autorisierten Person (z.B. dem Bewährungshelfer) einen direkten Zugriff auf die im Interlock-System gespeicherten Daten zu gewähren. Dieser Vorteil wird durch eine drahtlose Kommunikation zwischen dem Interlock-System und einem entfernt gelegenen Kontroll-System ermöglicht.

Ein weiterer Vorteil des erfindungsgemäßen Interlock-Systems besteht darin, dass die Weiterfahrt eines Fahrzeugs bei einem technischen Defekt des Interlock-Systems ermöglicht werden kann und/oder dass bestimmte Wartungsaufgaben am Interlock-System durchgeführt werden, ohne dafür eine Servicewerkstatt aufsuchen zu müssen. Wichtig hierbei ist es, dass zur Weiterfahrt bei Defekt des Interlock-Systems die Überbrückung der Interlock-Funktionalität nicht durch den Fahrer allein herbeigeführt werden kann, sondern dass mindestens eine freigebende autorisierte Person oder Institution beteiligt wird. Die Freigabe bzw. Deaktivierung des Interlock-Systems und dessen anschließende Reaktivierung erfolgt hierbei ebenfalls durch eine drahtlose Kommunikation zwischen dem Interlock-System und der autorisierten Person oder Institution, die Zugriff auf das Kontroll-System hat. Das Kontroll-System, oder allgemein Zielsystem, kann eine durch einen Computer zugängliche Datenbank oder einfach ein Mobiltelefon oder Faxgerät sein. Mit Hilfe der vorliegenden Erfindung wird ebenfalls erreicht, dass insbesondere die Freischaltfunktionalität im Falle eines defekten Interlock-Systems nicht Bestandteil des (potentiell defekten) Interlock-Systems ist, sondern dass diese Freischaltfunktionalität an das Interlock-System adaptiert wird.

Die vorliegende Erfindung wurde vorstehend allgemein für die Anwendung in einem Kraftfahrzeug beschrieben. Es soll jedoch verstanden werden, dass das erfindungsgemäße Interlock-System gleichermaßen dazu verwendet werden kann, um die Bedienung einer Maschine durch einen alkoholisierten Benutzer zu blockieren. Beispiele solcher Maschine sind große Baumaschinen, Maschinen in industriellen/chemischen Produktionsanlagen oder Kraftwerken, etc.

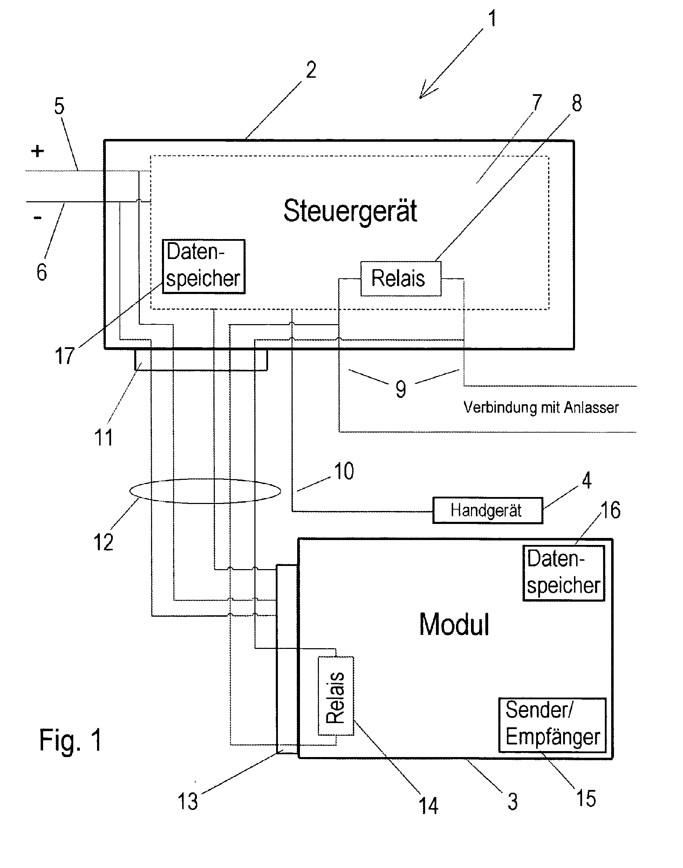

Die vorliegende Erfindung wird nun anhand eines Beispiels zur Anwendung in einem Kraftfahrzeug unter Bezugnahme auf Figur 1 beschrieben, die ein Blockdiagramm des erfindungsgemäßen Interlock-Systems zeigt.

Das in Figur 1 gezeigte Interlock-System 1 weist im Wesentlichen ein mit der Zündung eines Kraftfahrzeugs gekoppeltes Steuergerät 2, ein Modul 3 zur drahtlosen Datenübertragung und ein Handgerät 4 zur Alkoholmessung auf. Das Steuergerät 2 ist über zwei Anschlüsse 5, 6 an die Batterie (nicht gezeigt) des Fahrzeugs angeschlossen, in dem das Interlock-System 1 installiert ist. Die Komponenten des Steuergeräts sind in einem stabilen Gehäuse enthalten, das vorzugsweise unter dem Armaturenbrett des Fahrzeugs oder an einem anderen schwer zugänglichen und sicheren Bereich des Fahrzeugs montiert ist. Vorzugsweise sind alle wesentlichen Komponenten des Steuergeräts 2 auf einer Leiterplatte 7 vorgesehen. Zu diesen Komponenten gehört auch ein Relais 8 oder ein entsprechendes Schaltmittel (z.B. Thyristor, MOSFET, etc.), das über eine elektrische Verbindung 9 bevorzugt in den Stromkreis/Steuerungskreis des Anlassers (nicht gezeigt) zum Starten des Motors integriert ist. So kann zum Beispiel das Relais 8 in Form eines Ein/Aus-Schalters in Reihe zum Anlasser, zum Anlasserrelais, bzw. zu einer entsprechenden Komponente der Startelektronik geschaltet sein. Der Motor des Fahrzeugs lässt sich folglich nur dann starten, wenn das Relais 8 "eingeschaltet" ist, d.h. geschlossen ist. Wie vorstehend erwähnt, ist es für den Fachmann offensichtlich, dass anstelle des Relais 8 auch andere Schaltmittel vorgesehen sein können, wie zum Beispiel Leistungstransistoren, Thyristoren, MOSFETs, etc. Ferner ist es nicht zwingend erforderlich, dass das Relais 8 im Stromversorgungsschaltkreis des Anlassers bzw. des Anlasserrelais des Fahrzeugmotors integriert ist. Abhängig vom Typ des Motors und von der Art der Motorsteuerung sind auch andere Konfigurationen möglich. Entscheidend ist lediglich, dass mit Hilfe des Relais 8 oder eines entsprechenden Schaltmittels das Starten des Motors verhindert werden kann. So kann das Steuergerät 2 auch Teil der Startelektronik sein, wobei dann das Relais/Schaltmittel 8 durch ein relevantes Schalterelement der Startelektronik realisiert ist.

Außerdem ist ein Atemalkoholmessgerät 4 mit dem Steuergerät 2 elektrisch verbunden. Das Atemalkoholmessgerät 4 hat vorzugsweise die Form eines Handgeräts 4. Zum Beispiel kann das Handgerät ein "Dräger Interlock XT" Handgerät sein, das über ein Stromkabel bzw. Datenkabel 10 mit dem Steuergerät 2 verbunden ist. Die Verbindung kann jedoch auch drahtlos erfolgen, zum Beispiel über Infrarot, Bluetooth, etc. Das Handgerät 4 weist üblicherweise ein austauschbares Mundstück auf, das mit einem Alkoholsensor verbunden ist. Ferner können ein Display sowie mehrere Bedientasten vorgesehen sein. Der Alkoholsensor kann mit einer Auswerteelektronik verbunden sein, um aus der gemessenen Atemalkoholkonzentration den Alkoholpegel zu bestimmen.

Möchte ein Fahrer sein mit einem Interlock-System ausgestattetes Fahrzeug starten, so muss er zunächst mit Hilfe des Zündschlüssels die Zündung einschalten. Daraufhin erscheint auf dem Display des Handgeräts 4 (zusätzlich kann ein akustisches Signal ertönen) die Aufforderung, in das Mundstück des Handgeräts 4 zu pusten. Mit Hilfe des Alkoholsensors wird nun die Atemalkoholkonzentration bzw. der Alkoholpegel gemessen. Liegt dieser Alkoholpegel innerhalb des definierten Grenzbereichs, dann erfolgt die Freigabe, was durch eine Nachricht auf dem Display des Handgeräts angezeigt wird (auch hier kann zusätzlich ein akustisches Signal ertönen). Anschließend kann der Fahrer mit Hilfe des Zündschlüssels den Motor starten. Bei der Messung der Alkoholkonzentration/Alkoholpegel erzeugt der Alkoholsensor bzw. die Auswerteelektronik ein Signal, das vom Handgerät 4 über das Datenkabel 10 zum Steuergerät übertragen wird. Liegt die gemessene Atemalkoholkonzentration bzw. der Alkoholpegel innerhalb der definierten Grenzen, so generiert die Steuerelektronik (Auswerteeinheit) des Steuergeräts 2 ein entsprechendes Steuersignal zum "Einschalten" des Relais 8 im Zündschaltkreis zwischen Batterie und Anlasser. Liegt die gemessene Konzentration außerhalb dieser Grenzen, so bleibt das Relais 8 ausgeschaltet, wodurch ein Starten des Motors verhindert wird. Der definierte Grenzbereich, innerhalb dessen ein Starten des Motors zulässig ist, ist in einem Datenspeicher im Steuergerät selbst oder im Handgerät gespeichert und kann durch eine autorisierte Person bzw. durch eine autorisierte Institution fest eingestellt und natürlich auch verändert werden. Ferner werden alle relevanten Daten, d.h. Zeitpunkt des Startversuchs, gemessenen Atemalkoholkonzentration, mögliche Manipulationsversuche, etc., ebenfalls in einem Datenspeicher im Steuergerät 2 oder im Handgerät 4 gespeichert und können durch die autorisierte Person bzw. Institution ausgelesen werden. Das Eingeben bzw. Auslesen von Daten kann mit Hilfe eines Datenkabels, das am Steuergerät 2 oder am Handgerät 4 angeschlossen wird, oder auch drahtlos erfolgen, was später detailliert erläutert wird.

Wie in Figur 1 zu sehen, ist das Steuergerät 2 mit einer Schnittstelle versehen, die mit einem Datenstecker 11 gekoppelt werden kann. Der Datenstecker ist über ein Datenkabel 12 mit einem weiteren Datenstecker 13 verbunden, der mit einer Schnittstelle des Moduls 3 gekoppelt werden kann.

Das Modul 3 ist vorzugsweise ein vom Steuergerät 2 separates Bauelement, kann aber vorzugsweise mechanisch mit dem Gehäuse des Steuergeräts gekoppelt werden. Es ist ferner denkbar, dass die Schnittstellen des Steuergeräts bzw. des Moduls als männliche/weibliche Steckverbindungen ausgestaltet sind, so dass das Steuergerät und das Modul ohne Verwendung des Datenkabels und der Stecker direkt elektrisch miteinander gekoppelt werden können. Die Verbindung zwischen Steuergerät und Modul kann aber auch über eine oder mehrere Klemmen oder über ähnliche Verbindungsarten ausgeführt werden, um den Aufwand zur Installation in der Servicewerkstatt möglichst gering zu halten. Ferner ist es möglich, dass das Steuergerät 2 und das Modul 3 auf einer gemeinsamen Leiterplatte realisiert sind, die in einem "gemeinsamen" Gehäuse montiert ist. In diesem Fall erfolgt die Kopplung zwischen dem Handgerät 4 und dem Steuergerät/Modul 2, 3 vorzugsweise drahtlos, da das Gehäuse, in dem das Steuergerät 2 und das Modul 3 in integrierter Form enthalten sind, vorzugsweise fest im Inneren des Fahrzeugs montiert ist (beispielsweise unter dem Armaturenbrett oder im Motorraum).

Das Modul 3 enthält alle wesentlichem Komponenten für eine kabellose Datenübertragung von/zu einem nicht dargestellten, entfernt gelegenen Kontroll-System (zum Beispiel Computer/Server/Mobiltelefon einer autorisierten Person bzw. Institution) sowie ein zusätzliches Relais 14, um das Relais 8 des Steuergeräts 2 des Interlock-Systems 1 im Falle einer technischen Störung zu überbrücken. Die elektrische Verbindung (dargestellt als Stecker 11, 13 und Datenkabel 12) zwischen dem Steuergerät 2 und dem Modul 3 beinhaltet mindestens eine Spannungsversorgung, eine Datenleitung sowie zwei Kabel, mit Hilfe derer das Relais 14 zum Relais 8 des Steuergeräts parallel geschaltet wird, um das Relais 8 zu überbrücken.

Die Komponenten zur kabellosen Datenübertragung (zum Beispiel über UMTS, GSM, GPRS, etc.) sind in Figur 1 allgemein durch einen Block "Sender/Empfänger" 15 (nachfolgend kurz "Transceiver") dargestellt. Mit Hilfe des Transceiver-Moduls 3 bzw. des Transceivers 15 wird ein Auslesen eines Datenspeichers 16 im Transceiver-Modul 3 oder eines Datenspeichers 17 im Steuergerät 2 (komplett oder teilweise) ermöglicht, ohne dass dazu eine Servicewerkstatt aufgesucht werden muss. Der Datenspeicher 16 kann sich in dem Modul (wie dargestellt) oder im Steuergerät 2 (Datenspeicher 17) befinden, wobei im letztgenannten Fall die Datenübertragung vom Steuergerät 2 zum Modul 3 über die Datenleitung(en) des Datenkabels 12 erfolgt. Die zur Datenübertragung erforderlichen Kommunikationsprotokolle hängen von der verwendeten Art der Kommunikation (UMTS, GSM, GPRS, etc.) ab und sind dem Fachmann bekannt. Die Datenübertragung kann in regelmäßigen Abständen erfolgen, durch ein bestimmtes Ereignis (z.B. ein nicht bestandener Atemtest, eine bestimmte Anzahl von Atemtests oberhalb eines bestimmten Grenzwertes, ein vom Interlock registrierter Manipulationsversuch, der Ablauf einer Service- oder Kalibrierperiode, etc.) ausgelöst werden oder mit einem Signal (z.B. per SMS oder durch ein anderes geeignetes Protokoll) an das Interlock-System bzw. an das Modul 3 ausgelöst werden.

Die Daten werden dabei vom Speicher 16 oder 17 des Interlock-Systems an ein Zielsystem (Kontroll-System, Datenmanagement-System, etc.) übergeben. In diesem Zielsystem werden die Daten gespeichert und ggf. verarbeitet.

Unabhängig von der Übertragung der Daten aus dem Datenspeicher 16 oder 17 kann im Fall eines Fehlverhaltens des Fahrers (nicht bestandener Alkoholtest, Manipulationsversuch, etc.) eine Benachrichtigung vom Interlock-System 1 direkt an die verantwortliche Aufsichtspersonen (bzw. an die verantwortliche Institution) geschickt werden (z.B. an ein Mobiltelefon oder per Email/Fax).

Wie in Figur 1 gezeigt und vorstehend erläutert, ist das Modul 3 mit Hilfe eines Datenkabels 12 oder einer anderen geeigneten Verbindung mit dem Steuergerät 2 des Interlock-Systems 1 verbunden. Dabei erfolgt der Abgriff der Versorgungsspannung für das Modul 3 direkt an einer Stelle vor dem Abgriff der Versorgungsspannung für die Elektronik des Steuergeräts 2 an den Anschlüssen 5 und 6. Das bedeutet, dass bei dem gezeigten bevorzugten Ausführungsbeispiel das Steuergerät 2 des Interlock-Systems und das Modul 3 für die drahtlose Datenübertragung parallel miteinander verschaltet sind. Diese Parallelschaltung bewirkt eine vom Steuergerät unabhängige Spannungsversorgung des Moduls 3 und gewährleistet so eine Funktion des Moduls 3 auch bei einem vollständigen technischen Defekt des Steuergeräts 2.

Bei der gezeigten Ausführung des Interlock-Systems ist in Steuergerät 2 ein sogenanntes Freigaberelais 8 vorgesehen, das die Verbindung zwischen dem Zündschloss und dem Anlasser des Motors solange unterbricht, bis eine gültige Atemalkoholkonzentrationsmessung mit einem Messergebnis vorliegt, das die Freigabe des Startvorgangs erlaubt. Bei einem Defekt des Interlock-System 1, insbesondere des Steuergeräts 2, kann die Situation auftreten, dass das Relais 8 nicht mehr geschaltet werden kann und der Fahrer daher nicht mehr in der Lage ist, den Motor seines Fahrzeugs zu starten. Dieser Zustand wird mit dem beschriebenen "Sicherheitsrelais" 14 aufgehoben, das Teil des Moduls 3 ist und zum eigentlichen Freigaberelais 8 des Interlock-Systems parallel geschaltet ist.

Durch diese ODER-Schaltung der beiden Relais 8 und 14 wird es ermöglicht, ein blockierendes Interlock-System zu umgehen, indem das Sicherheitsrelais 14 geschlossen wird. Das Auslösen des Sicherheitsrelais 14 erfolgt dabei mittels drahtloser Datenübertragung zwischen dem Modul 3 und dem entfernt gelegenen Kontroll-System. Dabei sind zwei bevorzugte Ausführungen denkbar. In einer ersten Ausführung verfügt das Modul 3 über einen Sender/Empfänger (Transceiver) in Gestalt eines Mobiltelefon-Submoduls 15. Das Modul 3 ist somit in der Lage, beispielsweise eine SMS (Short Message Service) zu empfangen und den Absender dieser SMS sowie den Inhalt der SMS zu überprüfen. Der Fahrer des Fahrzeugs muss sich bei dieser Ausführungsform bei einer zur Freischaltung autorisierten Person/Instanz melden. Diese Instanz ist zum Beispiel der Bewährungshelfer, der Arbeitgeber oder eine andere Institution. Diese Instanz verschickt nun zum Beispiel über ein vorher benanntes Mobiltelefon eine SMS mit einem Freigabecode an das Mobiltelefon-Submodul 15 des Interlock-Moduls 3. Die im Modul 3 gespeicherte und ausführbare Software überprüft dann, ob der Freigabecode und das absendende Mobiltelefon autorisiert sind. Hierzu werden Verfahren verwendet, die dem Fachmann bekannt sind und hier nicht weiter erläutert werden. Das Modul 3 überbrückt anschließend bei erkannter und bestätigter Autorisierung des Freigabecodes mittels des Sicherheitsrelais 14 die Unterbrechung des Anlasser-Schaltkreises, indem das dem "defekten" bzw. geöffneten Freigaberelais 8 parallel geschaltete Sicherheitsrelais 14 geschlossen wird.

Sobald das Interlock-System, bzw. das Relais 8, mit Hilfe des Moduls 3, d.h. mit Hilfe des Relais 14, überbrückt wird, erfolgt (sofern der Defekt des Steuergeräts dies noch zulässt) über die Datenleitung zum Steuergerät des Interlock-Systems ein Eintrag in den Datenspeicher 17 des Interlock-Systems bzw. ein Eintrag im Datenspeicher 16, damit diese Überbrückung als "autorisierter Eingriff' registriert wird. Wie aus den vorstehenden Erläuterungen offensichtlich, können ein Datenspeicher 16 direkt im Modul 3 und/oder ein Datenspeicher 17 im Steuergerät 2 selbst vorgesehen sein.

In einer anderen bevorzugten Ausführung wird der Freigabecode mittels GPRS (General Packet Radio Service) übermittelt. Bei der Verwendung von GPRS ist die Menge der an einem Stück übermittelten Information potentiell größer, und daher können nicht nur ein praktisch beliebig langer Freigabecode sondern darüber hinaus weitere Informationen übermittelt werden. Diese Informationen umfassen unter anderem die zeitliche Befristung der Überbrückung.

Durch die drahtlose Datenübertragung wird ferner ermöglicht, eine "Fernwartung" durchführen zu können, d.h. Selbsttests des Interlock-Systems oder das Zurücksetzen von Timern oder Sperren (z.B. falls das Interlock nach Verstößen in einen "Lockout-Modus" versetzt wurde, der ein erneutes Starten des Fahrzeugs nicht mehr zulässt). Darüber hinaus können Servicedaten im Interlock-Datenspeicher 16 und/oder 17 sowie Parametereinstellungen (z.B. Grenzwerte, etc.) ausgelesen und überschrieben sowie Updates der Gerätesoftware durchgeführt werden.

Vorstehend wurde das Alkoholmessgerät 4 als Atemalkoholmessgerät beschrieben. Anstelle dieses Atemalkoholmessgeräts kann aber auch ein "transkutanes" Alkoholmessgerät verwendet werden, das an einem Körperteil der zu überwachenden Person befestigt wird. Mit Hilfe eines solchen Messgeräts kann eine kontinuierliche Kontrolle des Blutalkoholgehalts auch während des Betriebs des Fahrzeugs erfolgen. Dieses Messgerät kann dabei verdeckt am Körper getragen werden, zum Beispiel am Bein der Person, wo es durch die Hose verdeckt wird. Zweckmäßig ist es auch, das Messgerät am rechten Unterarm anzubringen. In vorteilhafter Weise ist zwischen dem transkutanen Alkoholmessgerät und dem Steuergerät, das die zugehörige Auswerteeinheit beinhaltet, eine drahtlose Übertragungsstrecke vorgesehen. Hierzu eignen sich Übertragungssysteme, die optisch, magnetisch oder mit Hochfrequenz arbeiten. Zweckmäßig ist es dabei, die Übertragungsstrecke bidirektional auszuführen, damit eine gegenseitige Datenkommunikation zwischen dem transkutanen Messgerät und der Auswerteeinheit des Interlock-Systems möglich ist.

## Patentansprüche

1. Interlock-System (1) für ein Fahrzeug, mit:
- einem Alkoholmessgerät (4), das ausgestaltet ist, um den Alkoholpegel eines Fahrers des Fahrzeugs zu messen;
- einem mit dem Alkoholmessgerät (4) gekoppelten Steuergerät (2), das ausgestaltet ist, um abhängig vom gemessenen Alkoholpegel das Starten des Fahrzeugs zu ermöglichen oder zu verhindern;
- einem mit dem Steuergerät (2) gekoppelten Transceiver-Modul (3), das ausgestaltet ist, um Daten zu einem entfernt von dem Steuergerät (2) gelegenen Kontroll-System drahtlos zu senden oder um Daten von diesem Kontroll-System drahtlos zu empfangen;
- wobei das Steuergerät (2) ein mit dem Anlasser des Fahrzeugs gekoppeltes erstes Schaltmittel (8) aufweist, das mittels einer Auswerteeinheit des Steuergeräts (2) abhängig von dem gemessenen Alkoholpegel geschaltet wird; **dadurch gekennzeichnet, dass**
- das Transceiver-Modul (3) ein zweites Schaltmittel (14) aufweist, das parallel zum ersten Schaltmittel (8) des Steuergeräts (2) geschaltet ist und
- das Transceiver-Modul (3) ferner dazu ausgestaltet ist, das zweite Schaltmittel (14) in Reaktion auf drahtlos empfangene Daten freizuschalten, um so unabhängig vom Betriebszustand des Steuergeräts (2) das Starten des Fahrzeugs zu ermöglichen.

2. Interlock-System (1) nach Anspruch 1, bei dem als erstes und / oder zweites Schaltmittel (8, 14) ein Relais, ein Halbleiter-Schaltmittel, wie Leistungstransistoren, Thyristoren oder MOSFETs, verwendet werden.

3. Interlock-System (1) nach einem der vorhergehenden Ansprüche, bei dem das Alkoholmessgerät (4) ein Atemalkoholmessgerät ist, das als ein Handgerät ausgestaltet und über ein elektrisches Kabel (10) mit dem Steuergerät (2) verbunden ist.

4. Interlock-System (1) nach einem der vorhergehenden Ansprüche, bei dem das Steuergerät (2) ausgestaltet ist, um abhängig von dem gemessenen Alkoholpegel die Stromzufuhr zu einem Anlasser des Fahrzeugs durchzuschalten oder zu blockieren.

5. Interlock-System (1) nach einem der vorhergehenden Ansprüche, bei dem das Transceiver-Modul (3) über ein elektrisches Kabel (12) mit dem Steuergerät (2) verbunden ist, wobei das Kabel mindestens eine Datenleitung, Spannungsversorgungsleitungen und Leitungen zum Überbrücken eines ersten Relais (8) aufweist, das in dem Steuergerät (2) vorgesehen und mit dem Anlasser des Fahrzeugs gekoppelt ist.

6. Interlock-System (1) nach einem der vorhergehenden Ansprüche, bei dem das Transceiver-Modul (3) unabhängig vom Steuergerät (2) mit Spannung versorgt wird, um das Steuergerät (2) im Fall eine technischen Defekts des Steuergeräts in Reaktion auf drahtlos übertragene Daten freischalten zu können.

7. Interlock-System (1) nach einem der vorhergehenden Ansprüche, bei dem das Transceiver-Modul (3) und/oder das Steuergerät (2) einen Datenspeicher (16, 17) aufweist, der mit Hilfe drahtloser Datenübertragung beschrieben und ausgelesen werden kann.

8. Interlock-System (1) nach einem der vorhergehenden Ansprüche, bei dem die durch das Transceiver-Modul (3) hervorgerufene Freischaltung zeitlich befristet ist oder die Dauer der Freischaltung dem Transceiver-Modul (3) mit Hilfe drahtlos übertragener Daten mitgeteilt wird.

9. Interlock-System (1) nach einem der vorhergehenden Ansprüche, bei dem mittels drahtloser Datenübertragung eine Wartung des Interlock-Systems und/oder ein Selbsttest des Interlock-Systems durchgeführt werden kann, wobei das Ergebnis des Selbsttests und/oder einzelne Prüfwerte drahtlos an das Kontroll-System übertragen werden.

10. Interlock-System (1) nach einem der vorhergehenden Ansprüche, bei dem mittels drahtloser Datenübertragung Parametereinstellungen des Interlock-Systems geändert und/oder Softwareupdates des Interlock-Systems durchgeführt werden können.

11. Interlock-System (1) nach einem der vorhergehenden Ansprüche, bei dem die drahtlose Datenübertragung in regelmäßigen Abständen erfolgt oder durch ein bestimmtes Ereignis ausgelöst wird.

12. Interlock-System (1) nach einem der vorhergehenden Ansprüche, bei dem das Steuergerät (2) und das Transceiver-Modul (3) auf einer gemeinsamen Leiterplatte realisiert sind.

13. Interlock-System (1) nach einem der vorhergehenden Ansprüche, bei dem das Transceiver-Modul (3) direkt mit dem Steuergerät (2) gekoppelt und mittels entsprechender Verbindungen mit diesem elektrisch verbunden ist.

## Claims

1. An interlock system (1) for a vehicle, comprising:
- an alcohol measuring device (4) adapted to measure an alcohol level of a driver of the vehicle;
- a control device (2) coupled to the alcohol measuring device (4) for allowing starting of the vehicle or preventing the vehicle from being started depending on the measured alcohol level;
- a transceiver module (3) coupled to said control device (2), which transceiver module is adapted for sending data to a remotely located control system in a wireless manner or for receiving data from this control system in a wireless manner;
- wherein said control device (2) has a switching means (8) coupled to the starter of the vehicle, said switching means being switched by means of an analysing unit of the control device (2) depending on the measured alcohol level; **characterized in that**
- said transceiver module (3) has a second switching means (14) connected in parallel to said fist switching means (8) of the control device (2), and
- said transceiver module (3) is further adapted to unlock the second switching means (14) in response to data received in a wireless manner in order to thereby allow starting of the vehicle regardless of the operating state of said control device (2).

2. An interlock system (1) according to claim 1, wherein a relay, a semiconductor switching means, such as power transistors, thyristors or MOSFETs, is used as said first and/or said second switching means (8, 14).

3. An interlock system (1) according to any of the preceding claims, wherein said alcohol measuring device (4) is a breath alcohol measuring device formed as a hand-held device and connected to said control device (2) via an electric cable (10).

4. An interlock system (1) according to any of the preceding claims, wherein said control device (2) is adapted to activate or block the current supply to a starter of the vehicle in dependence on the measured alcohol level.

5. An interlock system (1) according to any of the preceding claims, wherein said transceiver module (3) is connected to said control device (2) via an electric cable (12), wherein said electric cable has at least one data line, power supply lines and lines for bypassing a first relay (8) that is provided in the control device (2) and is coupled with the starter of the vehicle.

6. An interlock system (1) according to any of the preceding claims, wherein said transceiver module (3) is supplied with voltage independently from said control device (2) in order to be able to activate the control device (2) in case of a technical defect of the control device in response to data transmitted to said transceiver module in a wireless manner.

7. An interlock system (1) according to any of the preceding claims, wherein said transceiver module (3) and/or said control device (2) comprise a data memory (16, 17) written to and red by means of wireless data transmission.

8. An interlock system (1) according to any of the preceding claims, wherein the activation caused by said transceiver module (3) has a time limitation or the duration of the activation is reported to said transceiver module (3) by means of data transmitted in a wireless manner.

9. An interlock system (1) according to any of the preceding claims, wherein maintenance of the interlock system and/or a self-test of the interlock system can be performed by means of wireless data transmission, wherein the result of the self-test and/or individual checked values are transmitted to said control system in a wireless manner.

10. An interlock system (1) according to any of the preceding claims, wherein parameter settings of the interlock system can be changed and/or software updates of the interlock system can be performed by means of wireless data transmission.

11. An interlock system (1) according to any of the preceding claims, wherein said wireless data transmission takes place at regular intervals or is triggered by a particular event.

12. An interlock system (1) according to any of the preceding claims, wherein said control device (2) and said transceiver module (3) are embodied on a common printed circuit board.

13. An interlock system (1) according to any of the preceding claims, wherein said transceiver module (3) is coupled directly with said control device (2) and is electrically connected to said control device by means of corresponding connections.

## Revendications

1. Système de verrouillage (1) pour un véhicule avec :
- un appareil de mesure de l'alcool (4) qui est équipé pour mesurer l'alcoolémie du conducteur d'un véhicule ;
- un appareil de commande (2), couplé à l'appareil de mesure de l'alcool (4), qui est équipé pour permettre ou pour empêcher le démarrage du véhicule en fonction de l'alcoolémie mesurée ;
- un module émetteur-récepteur (3), couplé à l'appareil de commande (2), qui est équipé pour envoyer des données sans fil à un système de contrôle situé à distance de l'appareil de commande ou pour recevoir des données sans fil de ce système de contrôle,
- l'appareil de commande (2) présentant un premier moyen de commutation (8) couplé au démarreur du véhicule, moyen qui est mis en circuit au moyen d'une unité d'évaluation de l'appareil de commande (2) en fonction de l'alcoolémie mesurée,
**caractérisé en ce**
- **que** le module émetteur-récepteur (3) présente un second moyen de commutation (14) qui est commuté en parallèle avec le premier moyen de commutation (8) de l'appareil de commande (2) et
- **que** le module émetteur-récepteur (3) est de plus équipé pour débloquer le second moyen de commutation (14) en réaction à des données reçues sans fil pour permettre ainsi le démarrage du véhicule indépendamment de l'état de fonctionnement de l'appareil de commande (2).

2. Système de verrouillage (1) selon la revendication 1 pour lequel comme premier et/ou second moyen de commutation (8, 14) il est utilisé un relais, un moyen de commutation à semi-conducteur comme des transistors de puissance, des thyristors ou des transistors MOSFET.

3. Système de verrouillage (1) selon l'une des revendications précédentes pour lequel l'appareil de mesure de l'alcool (4) est un appareil de mesure respiratoire de l'alcool qui est configuré comme un appareil manuel et qui est relié à l'appareil de commande (2) par un câble électrique (10).

4. Système de verrouillage (1) selon l'une des revendications précédentes pour lequel l'appareil de commande (2) est configuré pour débloquer ou pour bloquer l'alimentation en courant du démarreur d'un véhicule en fonction de l'alcoolémie mesurée.

5. Système de verrouillage (1) selon l'une des revendications précédentes pour lequel le module émetteur-récepteur (3) est relié à l'appareil de commande (2) par un câble électrique (10), le câble présentant au moins une ligne de transmission de données, des conduites d'alimentation en courant et des conduites pour court-circuiter un premier relais (8) qui est prévu dans l'appareil de commande (2) et qui est couplé au démarreur du véhicule.

6. Système de verrouillage (1) selon l'une des revendications précédentes pour lequel le module émetteur-récepteur (3) est alimenté en courant indépendamment de l'appareil de commande (2) pour pouvoir débloquer l'appareil de commande (2) sur des données transmises sans fil en cas de défaillance technique de l'appareil de commande.

7. Système de verrouillage (1) selon l'une des revendications précédentes pour lequel le module émetteur-récepteur (3) et/ou l'appareil de commande (2) présente une mémoire de données (16, 17) qui peut être enregistrée et lue à l'aide d'une transmission de données sans fil.

8. Système de verrouillage (1) selon l'une des revendications précédentes pour lequel le déblocage provoqué par le module émetteur-récepteur (3) est limité dans le temps ou la durée du déblocage est communiquée au module émetteur-récepteur (3) à l'aide de données transmises sans fil.

9. Système de verrouillage (1) selon l'une des revendications précédentes pour lequel une maintenance du système de verrouillage et/ou un autocontrôle du système de verrouillage peut être effectué à l'aide d'une transmission de données sans fil, le résultat de l'autocontrôle et/ou des valeurs de contrôle individuelles étant transmises sans fil au système de contrôle.

10. Système de verrouillage (1) selon l'une des revendications précédentes pour lequel des réglages de paramètres du système de verrouillage peuvent être modifiés et/ou des actualisations de logiciel du système de verrouillage peuvent être effectuées à l'aide d'une transmission de données sans fil.

11. Système de verrouillage (1) selon l'une des revendications précédentes pour la transmission de données sans fil se fait à intervalles réguliers ou est déclenchée par un évènement précis.

12. Système de verrouillage (1) selon l'une des revendications précédentes pour lequel l'appareil de commande (2) et le module émetteur-récepteur (3) sont réalisés sur une plaquette de circuit imprimé commune.

13. Système de verrouillage (1) selon l'une des revendications précédentes pour lequel le module émetteur-récepteur (3) est couplé directement à l'appareil de commande (2) et est relié électriquement à celui-ci par des connexions correspondantes.
